# EUROPEAN PATENT APPLICATION

(11) **EP 2 695 942 A1**
(43) Date of publication of application: **12.02.2014**
(21) Application number: 12179587.6
(22) Date of filing: 07.08.2012
(51) Int. Cl.: C12N 15/113, A61K 31/7088, A61P 35/00

(54) **microRNAs in therapy and diagnostic of cancer**

(71) Applicant: Pelican Health Limited, London EC1M 4DN (GB)
(72) Inventor: The designation of the inventor has not yet been filed
(74) Representative: Jappy, John William Graham

(57) **Abstract**

The present invention provides an *ex vivo* method for determining whether tumour cells in a patient are, or are likely to become, resistant to a drug that modulates the binding of a steroid hormone to its receptor, comprising: (i) determining the expression level of one or more of the miRNAs listed in Table 1 and/or Table 2 in a tumour cell sample obtained from the patient; and (ii) comparing the expression level of each miRNA to a corresponding control (WT) value, wherein an increase in the cellular expression level of one or more miRNAs listed in Table 1 or a decrease in the cellular expression level of one or more of the miRNAs listed in Table 2 indicates that the tumour cells are, or are likely to become, resistant to the drug. Preferably the tumour cell is a breast cancer cell. Preferably the drug is a selective estrogen receptor modulator (SERM), preferably tamoxifen.

## Description

### Field of the Invention

The present invention relates to methods and therapeutic agents for preventing or reversing cellular drug resistance in patients suffering from a hormone-sensitive cancer, in particular breast cancer.

### Background of the Invention

Worldwide, more than a million women are diagnosed with breast cancer every year, accounting for a tenth of all new cancers and 23% of all female cancer cases. Breast cancer is the second most commonly diagnosed cancer among women, with a lifetime risk of about 13% and nearly 200,000 new cases diagnosed every year in the US alone. About 40,000 women in the US and 12,000 in the UK are expected to die from breast cancer in 2010, and breast cancer death rates are higher than those for any other cancer besides lung cancer.

As is the case with other solid malignancies, development and progression of breast cancer is a multi-step process involving genetic and epigenetic alterations that drive unrestrained cell proliferation and growth. Approximately 80% of breast cancers express a functional estrogen receptor (ER) and so are dependent on signalling by estrogen, a steroid hormone known to stimulate the growth of breast cells. Hence breast cancer is known as a hormone-sensitive cancer. Other hormone-sensitive cancers include prostate cancer.

The estrogen receptor (ER) is a member of the nuclear hormone family of intracellular receptors and its main function is as a ligand-activated DNA binding transcription factor regulating cell growth, differentiation and malignant transformation. There are two forms, ERα and β, each encoded by separate genes, ESR1 and ESR2 respectively; ERα is the major ER in mammary epithelium. Single-nucleotide polymorphisms of the ESR1 gene have been identified and are associated with different risks of developing breast cancer. ERs consist of six domains including the activation function (AF)-1 and AF-2 transcription activation domains. Their transcriptional activation is mediated by the synergistic action of ligand-independent AF-1 and ligand-regulated AF-2 and modified by nuclear receptor co-activators and co-repressors. Hormone binding to the receptor triggers a number of events starting with migration of the receptor from the cytosol into the nucleus, binding of homo- or hetero-dimers to estrogen response elements (ERE) in the promoters of estrogen-responsive genes and recruitment of transcription-associated proteins. The receptors also affect the activity of other transcription factor complexes such as AP-1 (Jun-Fos) and proteins that cooperate with ERα in transcriptional regulation include the p160 family (SRC-1, TIF2/SRC-2/GRIP1, AIB1/ACTR/SRC-3), cyclin D1 and several histone acetyl transferases (HATs including CBP, p300 and P/CAF. The ER has additional functions independent of DNA binding, with some ER associated with estrogenic regulation of signalling cascades, including the formation of complexes with G proteins as well as receptor and non-receptor tyrosine kinases, thus highlighting the cross-talk between estrogen and growth-factor-induced cytoplasmic signalling.

Since ER activation is the critical first step in tumourigenesis, all leading drugs used for endocrine therapy of ER-positive breast cancer interfere with estrogen binding to its receptor, thereby inhibiting the resulting growth stimulatory effects.

Tamoxifen has become the most widely used anti-estrogen drug since it was approved by the FDA for the treatment of metastatic breast cancer in 1977. As an adjuvant therapy in early breast cancer, tamoxifen improves overall survival and in previously untreated metastatic breast cancer more than 50% of patients with ER-positive tumours achieve an objective response. Tamoxifen is also believed to have clinical utility as a preventive agent for hormone-dependent breast cancer.

Tamoxifen is a non-steroidal cytostatic pro-drug that is metabolised in the liver by the cytochrome P450 isoform CYP2D6 and CYP3A4 into active metabolites such as 4-hydroxytamoxifen and 4-hydroxy-N-desmethyltamoxifen(endoxifen).These compete with estrogen binding to its receptor and inhibit the resulting growth stimulatory effects by maintaining cells in the G0 and G1 phases of the cell cycle.

However, the use of tamoxifen is limited by the onset of drug resistance. As a result, almost all patients with metastatic disease and as many as 40% of patients receiving adjuvant tamoxifen eventually relapse and die from their disease.

It is generally accepted that tamoxifen resistance occurs through a range of molecular changes involving irreversible genetic alterations of the cancer cells' DNA. Potentially reversible epigenetic changes have also been described, but these have been drug-induced and never been followed through clinically. In either case, these changes are thought to be selected from either an already heterogeneous population or arise from a two-stage process of cell alteration followed by cell selection due to the drug's cytostatic effect.

Other groups have previously postulated the concept of using drugs to alter epigenetics and reverse tamoxifen resistance, and a recent review states that miRNAs have a role in regulating chemoresistance and there are numerous potential therapeutic targets for reversing miRNA-mediated drug resistance (Kutanzi, K. R. et al. (2011) Clin Epigenetics 2:171-185).

The link between epigenetic alterations and Tamoxifen resistance was first reported 18 years ago (van Agthoven, T. et al. (1994) MolEndocrinol 8:1474-1483) and has been confirmed since (Sharma, D. et al., (2006) Cancer Res 66:6370-6378). When cells are exposed to histone deacytalase inhibitors, their levels of AKT are knocked down and, in the presence of Tamoxifen, the ability of the cells to proliferate is curtailed. Taking both drugs together can reverse tamoxifen resistance (Horiguchi, K. et al. (2002) Journal of Heart and Lung Transplantation 21:1090-1100).

MicroRNAs (miRNAs or miRs) are a class of short non-coding RNAs that regulate the expression of protein-coding genes at the post-transcriptional level by cleaving target mRNAs and/or repressing their translation. miRNAs are implicated in cancer and have been shown to act as oncogenes. miRNA-directed regulation of gene expression in human breast cancer (huBC) has been shown to influence its development and behaviour and aberrant miRNA expression is associated with different huBC sub-types.

It is now clear that there are extensive alterations in miRNA regulation associated with the estrogen signalling pathway. These play a key role in estrogen-dependent functions and highlight the importance of miRNA expression in the understanding of anti-estrogen resistance of breast cancer. A recent analysis of 453 miRNAs in 29 early-stage breast cancer tumours identified a predictive signature corresponding to ER status with 100% concordance compared to immunohistochemistry results (Lowery et al., 2009). However, another study profiled 208 miRNAs in 20 breast cancers and obtained an entirely different predictive signature of ER status (Mattie et al., 2006). This discrepancy could arise from differences in the clinicopathological parameters of the tumours, the use of different detection platforms or pre-analytical variables. Certainly this suggests that there is a significant amount of uncertainty about the details of specific miRNA involvement in estrogen-responsiveness in the published literature. Another study significantly linked four different miRNAs to ER+ breast cancer aggressiveness and one miRNA to metastatic capability in ER(-) cancer (Foekens et al., 2008).The expression of numerous miRNAs is extensively altered following estrogen treatment of cell lines and enforced expression of repressed miRNAs reduces estrogen-dependent cell growth (Maillot et al., 2009). However, the latter authors did not study tamoxifen-sensitivity.

A recent study by Ward et al (Oncogene (2012), 1-10) investigated the differential expression of miRNAs in tamoxifen resistant MCF7 cells compared with wild-type MCF7 cells, in the context of epithelial-mesenchymal transition (EMT).

The previous data on miRNA expression and drug resistance, including resistance to tamoxifen, need to be treated with caution because it has become clear that the main molecular techniques used in RNA analysis are significantly flawed and generate results that are often unreliable and misleading (Bustin et al., 2011; Bustin, 2008a; Bustin, 2005; Bustin, 2010; Bustin et al., 2005; Bustin and Dorudi, 2004; Bustin and Mueller, 2005; Bustin and Mueller, 2006; Bustin and Nolan, 2004; Bustin, 2004; Bustin, 2006; Bustin, 2008c; Bustin, 2008b).

There is a clear need for a greater understanding of the mechanisms of tamoxifen-resistance and improved treatment methodologies and regimes to prevent and/or reverse acquired resistance and therefore enhance the survival of patients with hormone-sensitive cancers.

### Summary of the Invention

According to a first embodiment, the present invention provides an *e*x *vivo* method for determining whether tumour cells in a patient are, or are likely to become, resistant to a drug that modulates the binding of a steroid hormone to its receptor, comprising: (i) determining the expression level of one or more of the miRNAs listed in Table 1 and/or Table 2 in a tumour cell sample obtained from the patient; and (ii) comparing the expression level of each miRNA to a corresponding control (WT) value, wherein an increase in the cellular expression level of one or more miRNAs listed in Table 1 or a decrease in the cellular expression level of one or more of the miRNAs listed in Table 2 indicates that the tumour cells are, or are likely to become, resistant to the drug. Preferably the tumour cell is a breast cancer cell. Preferably the drug is a selective estrogen receptor modulator (SERM), preferably tamoxifen.

In particular, an increase or decrease in cellular expression of at least +/-1.2-fold compared to the control is indicative of tamoxifen-resistance. The method according to the first aspect of the invention can be carried out to determine tamoxifen-resistance in tumour cells of a patient who has previously received tamoxifen therapy, or to determine the risk of a patient who has not yet received tamoxifen therapy becoming tamoxifen-resistant.

According to a second embodiment, the present invention provides an agent that decreases cellular expression of at least one miRNA listed in Table 1 or increases cellular expression of at least one miRNA listed in Table 2, for use in reverting tamoxifen-resistant tumour cells to a tamoxifen-sensitive phenotype for the treatment of breast cancer.

According to a third embodiment, the present invention provides a multicomponent therapeutic regimen for use in the treatment of a hormone-sensitive cancer, comprising the steps of:
(i) administering a first drug that modulates the binding of a steroid hormone to its receptor to a patient suffering from a hormone-sensitive cancer;
(ii) determining when the cancerous cells of the patient become, or are close to becoming, resistant to said first drug by carrying out the method according to any of claims 1 to 3;
(iii) administering a second drug, wherein the second drug is an agent that prevents DNA methylation and/or histone modification and/or decreases cellular expression of one or more of the miRNAs listed in Table 1 and/or increases cellular expression of one or more of the miRNAs listed in Table 2; and
(iv) ceasing to administer the second drug after a pre-determined period of time, or after establishing reversion to a drug-sensitive phenotype, wherein said regimen prevents the cancerous cells from becoming permanently resistant to said first drug.

According to a fourth embodiment, the present invention provides an *in vivo* method for reversing or preventing tamoxifen resistance in a tumour cell, comprising administering to a patient one or more agents that lead to an increase in cellular expression of one or more miRNAs that are down-regulated in tamoxifen-resistant (TR) cells, or lead to decrease in cellular expression of miRNAs that are up-regulated in TR cells.

According to a fifth embodiment, the invention provides an *e*x *vivo* method for determining whether tumour cells previously identified as being resistant to a drug that modulates the binding of a steroid hormone to its receptor have reverted to a drug-sensitive phenotype, comprising (i) identifying one or more of the miRNAs listed in Table 1 and/or Table 2 that was previously known to be over- or under-expressed in a tumour cell obtained from a patient having a drug-resistant phenotype; (ii) determining the expression level of the one or more of the miRNAs identified in step (i) in a tumour cell sample obtained from the patient; and either (iii) comparing the expression level to a corresponding control (WT) value, wherein a value within less than +/-1.2-fold of the control value indicates that the cell has reverted to a drug-sensitive phenotype; or (iv) comparing the expression level to the expression level of the corresponding miRNA as previously measured in the patient sample known to be drug-resistant, wherein a decrease of at least 1.2-fold, in the cellular expression level of one or more miRNAs listed in Table 1 and/or an increase of at least 1.2-fold,inthe cellular expression level of one or more of the miRNAs listed in Table 2 indicates that the tumour cells have reverted from a resistant to sensitive phenotype. The drug is preferably a selective estrogen receptor modulator (SERM), preferably tamoxifen.

According to a sixth embodiment, the invention provides a biochip which can detect the expression level of one or more miRNAs implicated in tamoxifen-resistance. The biochip can be used to determine whether tumour cells in a patient sample have become, or are likely to become, resistant to tamoxifen therapy, or to determine whether previously resistant tumour cells have reverted to a tamoxifen-sensitive phenotype.

According to a seventh embodiment, the invention provides an *in vitro* method for restoring a tamoxifen-sensitive phenotype in cells of a tamoxifen-resistant cell line, comprising repeatedly re-incubating the cells in the absence of tamoxifen.

### Description of the Drawings

Figure 1 is a graph showing the effect of high dose tamoxifen (10⁻⁷M) on the growth of wild-type (WT) and tamoxifen-resistant (TR) breast cancer cells over a 15 day period;
Figure 2 is a graph showing that TR cells show loss of the normal stimulatory response to estradiol;
Figure 3 is a graph showing the effects of high dose tamoxifen (10⁻⁷M) on growth of a Tam-S revertant cell line(TRev), WT and TR cells (mean +/-SD of five experiments).

### Detailed Description of the Invention

The present invention is based upon the surprising discovery that resistance to drugs that modulate the binding of a steroid hormone to its receptor, such as tamoxifen, which was previously believed to be an exclusively irreversible process, can be a reversible phenomenon in individual cells resulting from the molecular events relating to epigenetic, rather than genetic, alterations. By targeting these molecular events, resistance to such drugs can be reversed or prevented in patients receiving drug treatment. Furthermore, the present inventors have shown that Tam-resistant cells can revert to Tam-sensitivity without the need to add any drug to up- or down-regulate any mRNA or use miRNA mimics or antagonists.

The inventors have identified a number of miRNAs (also referred to herein as miRs) that are either up-regulated or down-regulated in resistant cells by at least +/- 1.2-fold compared with wild-type (WT) control cells and it has been shown that miRNAs which are up-regulated in tamoxifen-resistant (TR) cells have expression levels which revert back to towards those of WT cells in the tamoxifen-revertant cells. Similarly miRNAs which were down-regulated in the TR cells increased back towards normal in the tamoxifen-revertant cells. The protocol detailed in Example 1 can be used to determine whether the threshold of at least+/- 1.2-foldcompared with wild-type is met for a given miRNA.

In the context of the present invention, it is preferred that the drug that modulates the binding of a steroid hormone to its receptor is a Selective Estrogen Receptor Modulator (SERM). SERMs are a class of compounds that act on the estrogen receptor (ER). This class includes the drug tamoxifen.

Tamoxifen is the generic name for the compound (Z)-2-[4-(1,2-diphenylbut-1-enyl)phenoxy]-N,N-dimethyl-ethanamine that is marketed under several brand names including Nolvadex™, Istubal™ and Valodex™ for the treatment of breast cancer. Furthermore, the observed correlation between the relative expression levels of certain miRNAs and drug-resistance can be used to determine the risk of a patient who has not yet been treated with an anti-cancer therapy becoming resistant to said therapy. This information is extremely useful to clinicians; if a patient is identified as being susceptible to becoming tamoxifen-resistant, they can take steps to mitigate the risk, such as administering agents according to the present invention, as described below. As a result, the efficacy of the cancer therapy is improved.

Other compounds within this class include clomifene (2-(4-(2-chloro-1,2-diphenylethenyl)phenoxy)-N,N-diethyl-ethanamine), which is used for ovarian stimulation in female infertility, femarelle (used to treat symptoms of menopause), ormeloxifene (used in oral contraceptives), raloxifene, toremifene, afimoxifene and bazedoxifen.

Compounds within the SERM class can function either as agonists or antagonists of the estrogen receptor (ER), and they can have a different action in different tissues. Therefore, a member of this class could stimulate the ER in one tissue and inhibit it in another. As such, SERMs are used according to their pattern of action in various tissues.

Tamoxifen itself is non-steroidal pro-drug, having relatively little affinity for its target protein, the estrogen receptor. However, it is metabolized in the liver by the cytochrome P450 isoforms CYP2D6 and CYP3A4 into active metabolites, such as 4-hydroxytamoxifen and N-desmethyl-4-hydroxytamoxifen, which have 30-100 times more affinity for the estrogen receptor than tamoxifen itself and compete with estrogen for binding to the estrogen receptor. Tamoxifen causes cells to remain in the Go and G₁ phases of the cell cycle. Because it prevents cells from dividing, but does not cause cell death, tamoxifen is a cytostatic, rather than cytocidal, drug.

It is also within the scope of the present invention to reverse or prevent cellular resistance to other drugs that bind competitively to the estrogen receptor.

According to a first embodiment, the present invention provides an *ex vivo* method for determining whether tumour cells in a patient are, or are likely to become, resistant to a drug that modulates the binding of a steroid hormone to its receptor, comprising: (i) determining the expression level of one or more of the miRNAs listed in Table 1 and/or Table 2 in a tumour cell sample obtained from the patient; and (ii) comparing the expression level of each miRNA to a corresponding control (WT) value, wherein an increase in the cellular expression level of one or more miRNAs listed in Table 1 or a decrease in the cellular expression level of one or more of the miRNAs listed in Table 2 indicates that the tumour cells are, or are likely to become, resistant to the drug. Preferably the tumour cell is a breast cancer cell. Preferably the drug is a selective estrogen receptor modulator (SERM), preferably tamoxifen.

Preferably, the expression level of at least five, and more preferably five to ten, of the miRNAs listed in Table 1 and/or Table 2 is measured and compared with corresponding control (WT) values.

Table 1 is a list of miRNAs that are up-regulated in tamoxifen-resistant (TR) cells. Table 2is a list of miRNAs that are down-regulated in TR cells. The fold-difference in expression level of each miRNA in TR and WT cells is also shown in each table.

For the avoidance of doubt, "hsa-" refers to *homo sapiens* and is a standard abbreviation to differentiate the miRNAs from those of other species. The suffixes "3p" and "5p" denote 3 prime or 5 prime, respectively. These suffixes are used to distinguish two miRNAs originating from opposite arms of the same pre-miRNA. "let-7" refers to the lethal-7 gene, which is a miRNA precursor.

All miRNAs are identified herein using standard nomenclature. Sequence information for each of the miRNAs listed in Tables 1 and 2 can be found on the miRBase database maintained by Manchester University (http://mirbase.org/search.shtml).

**Table 1**

| **miRNA** | **Relative Expression TR vs WT** |
|---|---|
| hsa-miR-21 | 21.6 |
| hsa-miR-16 | 7.1 |
| hsa-miR-26a | 5.4 |
| hsa-miR-141 | 3.2 |
| hsa-miR-15a | 3.1 |
| hsa-miR-15b | 3.1 |
| hsa-miR-24 | 2.6 |
| hsa-miR-26b | 2.5 |
| hsa-miR-103 | 2.3 |
| hsa-miR-191 | 2.2 |
| hsa-miR-520d-5p | 2.2 |
| hsa-miR-527 | 2.2 |
| hsa-miR-518a-5p | 2.2 |
| hsa-miR-30b | 2.2 |
| hsa-miR-30c | 2.2 |
| hsa-miR-200a | 2.1 |
| hsa-miR-361-5p | 2.0 |
| hsa-miR-770-5p | 2.0 |
| hsa-miR-106b | 1.9 |
| hsa-miR-96 | 1.9 |
| hsa-miR-30d | 1.5 |
| hsa-miR-301a | 1.5 |
| hsa-miR-425 | 1.3 |
| hsa-miR-455-3p | 1.3 |
| hsa-miR-374b | 1.2 |
| hsa-miR-454 | 1.2 |

**Table 2**

| **miRNA** | **Relative Expression TR:WT** |
|---|---|
| hsa-miR-205 | -5.2 |
| hsa-miR-193a-3p | -5.0 |
| hsa-miR-542-3p | -4.2 |
| hsa-let-7d | -1.2 |
| hsa-let-7a | -1.2 |
| hsa-miR-548h | -1.2 |
| hsa-miR-365 | -1.2 |
| hsa-miR-93 | -1.3 |
| hsa-miR-620 | -1.3 |
| hsa-miR-548j | -1.3 |
| hsa-miR-31 | -1.3 |
| hsa-miR-181b | -1.3 |
| hsa-miR-181d | -1.3 |
| hsa-miR-450a | -1.3 |
| hsa-miR-302f | -1.3 |
| hsa-let-7c | -1.4 |
| hsa-miR-660 | -1.4 |
| hsa-miR-218 | -1.4 |
| hsa-miR-188-5p | -1.4 |
| hsa-miR-892a | -1.4 |
| hsa-miR-320a | -1.4 |
| hsa-miR-720 | -1.4 |
| hsa-miR-181a | -1.5 |
| hsa-miR-645 | -1.5 |
| hsa-miR-572 | -1.5 |
| hsa-miR-518f | -1.6 |
| hsa-miR-1289 | -1.6 |
| hsa-miR-649 | -1.6 |
| hsa-miR-324-5p | -1.6 |
| hsa-miR-1299 | -1.6 |
| hsa-miR-378 | -1.6 |
| hsa-miR-335 | -1.6 |
| hsa-miR-592 | -1.6 |
| hsa-miR-302c | -1.7 |
| hsa-miR-526a | -1.7 |
| hsa-miR-518d-5p | -1.7 |
| hsa-miR-520c-5p | -1.7 |
| hsa-miR-1279 | -1.7 |
| hsa-miR-1182 | -1.7 |
| hsa-miR-503 | -1.7 |
| hsa-miR-222 | -1.8 |
| hsa-miR-1283 | -1.8 |
| hsa-miR-320d | -1.8 |
| hsa-miR-555 | -1.8 |
| hsa-miR-1537 | -1.8 |
| hsa-miR-664 | -1.8 |
| hsa-miR-519e | -1.8 |
| hsa-miR-136 | -1.8 |
| hsa-miR-580 | -1.8 |
| hsa-miR-492 | -1.8 |
| hsa-miR-495 | -1.8 |
| hsa-miR-525-3p | -1.9 |
| hsa-miR-383 | -1.9 |
| hsa-miR-615-5p | -1.9 |
| hsa-miR-875-3p | -1.9 |
| hsa-miR-488 | -1.9 |
| hsa-miR-1298 | -1.9 |
| hsa-miR-450b-5p | -1.9 |
| hsa-miR-484 | -1.9 |
| hsa-miR-876-5p | -1.9 |
| hsa-miR-363 | -1.9 |
| hsa-miR-1909 | -1.9 |
| hsa-miR-514 | -1.9 |
| hsa-miR-655 | -1.9 |
| hsa-miR-1277 | -1.9 |
| hsa-miR-1973 | -1.9 |
| hsa-miR-34c-3p | -2.0 |
| hsa-miR-142-5p | -2.0 |
| hsa-miR-410 | -2.0 |
| hsa-miR-2115 | -2.0 |
| hsa-miR-515-3p | -2.0 |
| hsa-miR-661 | -2.0 |
| hsa-miR-548g | -2.0 |
| hsa-miR-767-5p | -2.0 |
| hsa-miR-412 | -2.0 |
| hsa-miR-146a | -2.0 |
| hsa-miR-574-5p | -2.1 |
| hsa-miR-490-5p | -2.1 |
| hsa-miR-362-5p | -2.1 |
| hsa-miR-641 | -2.1 |
| hsa-miR-548f | -2.1 |
| hsa-miR-328 | -2.1 |
| hsa-miR-663 | -2.1 |
| hsa-miR-337-5p | -2.1 |
| hsa-miR-1321 | -2.1 |
| hsa-miR-208b | -2.1 |
| hsa-miR-10b | -2.1 |
| hsa-miR-708 | -2.1 |
| hsa-miR-491-3p | -2.1 |
| hsa-miR-619 | -2.2 |
| hsa-miR-375 | -2.2 |
| hsa-miR-190b | -2.2 |
| hsa-miR-1206 | -2.2 |
| hsa-miR-423-5p | -2.2 |
| hsa-miR-566 | -2.2 |
| hsa-miR-193a-5p | -2.2 |
| hsa-miR-453 | -2.2 |
| hsa-miR-302b | -2.2 |
| hsa-miR-496 | -2.2 |
| hsa-miR-623 | -2.2 |
| hsa-miR-99b | -2.2 |
| hsa-miR-590-3p | -2.2 |
| hsa-miR-1287 | -2.2 |
| hsa-miR-1268 | -2.3 |
| hsa-miR-553 | -2.3 |
| hsa-miR-651 | -2.3 |
| hsa-miR-608 | -2.3 |
| hsa-miR-125a-5p | -2.3 |
| hsa-miR-1979 | -2.3 |
| hsa-miR-34b | -2.3 |
| hsa-miR-208a | -2.3 |
| hsa-miR-7 | -2.3 |
| hsa-miR-206 | -2.4 |
| hsa-miR-561 | -2.4 |
| hsa-miR-609 | -2.4 |
| hsa-miR-576-5p | -2.4 |
| hsa-miR-197 | -2.5 |
| hsa-miR-1308 | -2.5 |
| hsa-miR-33a | -2.5 |
| hsa-miR-127-5p | -2.5 |
| hsa-miR-635 | -2.5 |
| hsa-miR-548c-5p | -2.5 |
| hsa-miR-1915 | -2.6 |
| hsa-miR-499-5p | -2.6 |
| hsa-miR-760 | -2.6 |
| hsa-miR-510 | -2.6 |
| hsa-miR-600 | -2.7 |
| hsa-miR-199a-3p | -2.7 |
| hsa-miR-199b-3p | -2.7 |
| hsa-miR-554 | -2.7 |
| hsa-miR-938 | -2.8 |
| hsa-miR-137 | -2.9 |
| hsa-miR-629 | -3.0 |
| hsa-miR-603 | -3.0 |
| hsa-miR-769-5p | -3.0 |
| hsa-miR-1825 | -3.1 |
| hsa-let-7e | -3.1 |
| hsa-miR-1255a | -3.3 |
| hsa-miR-595 | -3.3 |
| hsa-miR-1908 | -3.4 |
| hsa-miR-216a | -3.5 |
| hsa-let-7b | -3.6 |

Nanostring Technologies have shown that an increase or decrease in cellular expression measured by their technology of more than 1.2-fold is of statistical significance. For the purpose of the present invention, the increase or decrease in cellular expression of the miRNA is at least 1.2-fold in TR cells compared to the corresponding WT control. This is indicative of tamoxifen-resistance.

Nanostring technology has not previously been used to study miRNA expression. Most de-novo experiments are carried out using microarrays. This involves hybridisation of thousands of nucleic acids at a single annealing temperature, which is suboptimal and accounts for much of the variation seen with this technology. Moreover, the miRNA microarray method measures the miRNA precursor as well as the mature miRNA and the procedure is not suitable for discriminating between miRNA species of high similarity, leading to false positive results. RT-qPCR is both more sensitive and more accurate and comparisons between microarray and RT-qPCR have demonstrated superior sensitivity and specificity of RT-qPCR, as well as low correlation with microarrays(r = -0.443). However, the inventors have found that the Nanostring method is advantageous over the qPCR method for studying miRNA expression, for a number of reasons. Firstly, the Nanostring method incorporates only one enzymatic step in its workflow and is therefore less prone to technical bias than the qPCR-based protocols that incorporate three enzymatic steps. Secondly, the Nanostring technique measures every single miRNA at exactly the same time in a single reaction vessel, which reduces the miRNA to miRNA bias. Thirdly, Nanostring uses digital detection: brightness of the probe does not carry any information; a target is simply recorded as present or not present. Hence a Nanostring result is the number of times that a RNA was counted in that sample. In contrast, qPCR relies on the relative levels of a fluorescent signal for detection and quantification. The relative fluorescence of the PCR varies with different probes, reaction mixtures and instruments, and depends on the efficiency of conversion of RNA to DNA, the PCR efficiency, the choice of reference genes and the chosen quantification cycle cut-off level. That cut-off level is not an absolute and since normalisation to reference genes is a critical factor in qPCR-based data analysis, the choice of reference gene determines what results are recorded. This introduces significant experiment-to-experiment variability and is a well-described and critical factor underlying the significant lack of concordance in the literature between different laboratories. Due to these differences, the Nanostring system can measure as little as 1.2-fold changes of a single transcript at 20 copies per cell (10fM) with statistical significance (p<0.05). The Coefficient of Variation in the one copy per cell expression range is ∼10% and even lower for more highly expressed miRNAs.

If the method of the invention is carried out using a sample obtained from a patient who has previously been treated using tamoxifen, then the method can be used to determine whether the tumour cells have become or are likely to become resistant to tamoxifen. Alternatively, the method can be carried out prior to administering anti-cancer therapy, such as tamoxifen, to the patient. In this way, the method of the invention enables a clinician to determine whether the tumour cells are likely to become tamoxifen-resistant. If so, the subsequently administered anti-cancer therapy may involve administering tamoxifen together with or in addition to one or more agents that modulate the expression of miRNAs identified as being over- or under-expressed relative to the controls.

Alternatively, based on the results obtained by the method of the invention, the clinician may decide that the patient will not be responsive to tamoxifen due to the likelihood of developing tamoxifen-resistance and as such may prescribe an alternative anti-cancer therapy.

The terms "control" and "wild-type (WT)" are used interchangeably. For each of the miRNAs listed in Tables 1 and 2, a corresponding control value will be pre-determined. This control value indicates an expression level for the respective miRNA, as measured in healthy wild-type cells, preferably of the same cell type as the tumour, preferably breast tissue cells.

"TR" is an abbreviation for tamoxifen-resistant cells or cell lines. "TRev" is an abbreviation for tamoxifen-sensitive revertant cells or cell lines (i.e. cells or cell lines that were previously resistant to tamoxifen but have been reverted to a tamoxifen-sensitive phenotype).

The terms "cancer" and "tumour" are used interchangeably. Preferably, the cancer is a hormone-sensitive cancer, preferably breast cancer, and the tumour cell is a breast cancer cell.

As used herein, the term 'patient' refers to a mammal including a non-primate (e.g. a cow, pig, horse, dog, cat, rat and mouse) and a primate (e.g. a monkey and human), and preferably a human.

According to a second embodiment, the invention provides an agent that decreases cellular expression of at least one miRNA that has been found to be over-expressed in TR calls, preferably at least one of the miRNAs listed in Table 1, or an agent that increases cellular expression of at least one miRNA that has been found to be under-expressed in TR cells, preferably at least one of the miRNAs listed in Table 2, for use in reverting tamoxifien-resistant tumour cells to a tamoxifen-sensitive phenotype for the treatment of breast cancer.

Preferably the agent is selected from the group comprising miRNA or anti-miRNA molecules and drugs which prevent DNA methylation and/or histone modification, such as 5'-modified analogs of deoxycytidine, 5-azacytidine (5-azaC, azacitidine; Vidaza™), 5-aza-2'-deoxycytidine,zebularine, 5-fluoro-2'-deoxycytidine,butyrates (such as phenylbutyrate and sodium butyrate), valproic acid, suberoylanilidehydroxamic acid (vorinostat; Zolinza™), and dispeptide, non-nucleoside weak inhibitors, such as procainamide and hydralazine and (rationally designed inhibitors of DNA methylation, including small molecule inhibitors of DNA methyltransferases (DMTF). These pharmaceutically-acceptable agents fall within the scope of the invention.

In a further embodiment, the present invention provides a therapeutic regimen for use in the treatment of a hormone-sensitive cancer, comprising the steps of:
(i) administering a first drug that modulates the binding of a steroid hormone to its receptor to a patient suffering from a hormone-sensitive cancer;
(ii) determining when the cancerous cells of the patient become, or are close to becoming, resistant to said first drug by carrying out the method according to the first embodiment of the invention;
(iii) administering a second drug, wherein the second drug is an agent that prevents DNA methylation and/or histone modification and/or decreases cellular expression of one or more of the miRNAs listed in Table 1 and/or increases cellular expression of one or more of the miRNAs listed in Table 2; and
(iv) ceasing to administer the second drug after a pre-determined period of time, or after establishing reversion to a drug-sensitive phenotype, wherein said regimen prevents the cancerous cells from becoming permanently resistant to said first drug.

Preferably, the steroid hormone is estrogen and the receptor is the estrogen receptor. Preferably the first drug is a selective estrogen receptor modulator, preferably tamoxifen.

Therapy with the first drug (i.e. the drug that modulates the binding of a steroid hormone to its receptor) can optionally be withdrawn from the patient whilst the second drug is administered and subsequently re-administered, once administration of the second drug has ceased.

The second drug is preferably selected from the group comprising miRNA or anti-miRNA molecules and drugs which prevent DNA methylation and/or histone modification; multiple drugs of this type may be administered, either separately or in combination. Suitable drugs for preventing DNA methylation and/or histone modification may be selected from 5-azacytidine, 5-aza-2'-deoxycytidine, zebularine, 5-fluoro-2'-deoxycytidine, butyrates (such as phenylbutyrate and sodium butyrate), suberoylanilidehydroxamic acid and valproic acid.

It is within the scope of the present invention for the second drug to comprise a combination of epigenetic therapies, i.e. a combination of a DMTF inhibitor and a HDAC inhibitor. Examples of suitable combination therapies on include (i) 5-azacytidine and phenylbutyrate, and (ii) decitabine and valproic acid.

The first and second drugs may be administered via any suitable route, such as oral or intravenous administration routes.

The method according to the fifth embodiment of the present invention may be used to determine when the cells have reverted from a drug-resistant to drug-sensitive phenotype. At this point, administration of the second drug can cease and treatment with the first drug can resume.

According to a further embodiment, the present invention provides an *in vivo* method for reversing or preventing tamoxifen resistance in a tumour cell, comprising administering to a patient one or more agents that lead to an increase in cellular expression of one or more miRNAs that are down-regulated in TR cells, or lead to decrease in cellular expression of miRNAs that are up-regulated in TR cells.

Preferably, the one or more miRNAs that are down-regulated in TR cells will need to be up-regulated and are selected from the miRNAs listed in Table 2, and/or wherein one or more miRNAs that are up-regulated in TR cells will need to be down-regulated and are selected from the miRNAs listed in Table 1.

Preferably, the one or more agents are selected from the group comprising miRNA or anti-miRNA molecules and drugs which prevent DNA methylation and/or histone modification; these may be administered either separately or in combination and may be selected from including 5-azacytidine, 5-aza-2'-deoxycytidine, zebularine, 5-fluoro-2'-deoxycytidine, butyrates (such as phenylbutyrate and sodium butyrate), suberoylanilidehydroxamic acid and valproic acid. According to the method of the invention, two or more agents may be administered separately or in combination, and either simultaneously or consecutively.

According to a fifth embodiment, the invention provides an *ex vivo* method for determining whether tumour cells previously identified as being resistant to a drug that modulates the binding of a steroid hormone to its receptor have reverted to a drug-sensitive phenotype, comprising (i) identifying one or more of the miRNAs listed in Table 1 and/or Table 2 that was previously known to be over- or under-expressed in a tumour cell obtained from a patient having a drug-resistant phenotype; (ii) determining the expression level of the one or more of the miRNAs identified in step (i) in a tumour cell sample obtained from the patient; and either (iii) comparing the expression level to a corresponding control (WT) value, wherein a value within less than +/-1.2-fold of the control value indicates that the cell has reverted to a drug-sensitive phenotype; or (iv) comparing the expression level to the expression level of the corresponding miRNA as previously measured in the patient sample known to be drug-resistant, wherein a decrease of at least 1.2-fold, preferably at least 1.5-fold, or more preferably at least 1.7-fold, in the cellular expression level of one or more miRNAs listed in Table 1 and/or an increase of at least 1.2-fold,preferablyat least 1.5-fold, or more preferably at least 1.7-fold, in the cellular expression level of one or more of the miRNAs listed in Table 2 indicates that the tumour cells have reverted from a resistant to sensitive phenotype, wherein the drug is preferably a selective estrogen receptor modulator (SERM), preferably tamoxifen.

In a further embodiment, the present invention provides a biochip for determining the expression level of one of more of the miRNAs listed in Table 1 and/or Table 2 present in a patient sample. The biochip comprises oligonucleotide sequences specific to each of the one or more miRNA molecules. Preferably, the biochip can detect five or more of the miRNAs listed in Tables 1 and/or 2, more preferably up to ten, more preferably up to twenty, and most preferably all of the miRNA molecules listed in the tables.

The biochip of the invention can be used to determine whether tumour cells in a patient sample have become, or are likely to become, resistant to tamoxifen therapy and can also be used to determine whether previously resistant tumour cells have reverted to a tamoxifen-sensitive phenotype, for example, as a result of the therapeutic methods and regimens encompassed by the present invention. The biochip provides expression level values for the miRNAs present in a patient sample; these values can be compared with corresponding control (WT) values in order to determine with the miRNAs are over- or under- expressed in the patient sample.

According to a further embodiment, the present invention provides an *in vitro* method for restoring a tamoxifen-sensitive phenotype in cells of a tamoxifen-resistant cell line.

The method comprises repeatedly re-incubating the cells in the absence of tamoxifen. The tamoxifen-resistant cell line is preferably a hormone-sensitive cancer cell line, particularly a breast cancer cell line that is derived from a tamoxifen-sensitive cell line, such as the MCF-7 cell line (Accession No. ATCC HTB-22).

A tamoxifen-resistant cell line according to the present invention has been deposited at the European Collection of Cell Cultures, HPA Culture Collections, Health Protection Agency, Porton Down, Salisbury, Wiltshire SP4 OJG United Kingdom and has been accorded the Accession No. ECACC 11050502.

According to the method, the cells are repeatedly re-incubated for a period of at least six months, preferably nine months.

It is preferred that the cells are re-incubated in the presence of estrogen, preferably at a concentration of 10⁻⁹M.

A tamoxifen-sensitive cell line can be obtained by the method and such a cell line is suitable for use in therapy, particularly for the treatment of cancer, in particular, breast cancer.

Tamoxifen-sensitive cells obtainable by the above-described method are also suitable for use in an *ex vivo* assay to determine when estrogen receptors in a sample from a patient receiving tamoxifen therapy switch from a tamoxifen-sensitive phenotype to a tamoxifen-resistant phenotype.

A tamoxifen-sensitive cell line can be obtained by method and such a cell line is suitable for use in therapy, particularly for the treatment of cancer, in particular, breast cancer.

A tamoxifen-sensitive cell line (i.e. a revertant cell line, termed TRev) according to the present invention has been deposited at the European Collection of Cell Cultures, HPA Culture Collections, Health Protection Agency, Porton Down, Salisbury, Wiltshire SP4 OJG United Kingdom and has been accorded the Accession No. ECACC 11050501.

The present invention will now be exemplified by reference to the following non-limiting examples.

### Example 1

Protocol for selecting miRNAs of the invention:
1. The Nanostring data on all the miRNAs provides an initial raw count of detection for each miRNA.
2. Positive controls are used to normalise the raw data. Processing can introduce variation, therefore each experiment contains a set of positive controls made up of a single miRNA 'spike' control which is annealed, ligated and purified with its own unique tag in the reaction tubes along with the sample miRNA. Each experiment includes its own spike, which is included at six different concentrations ranging from 0.125fM to 128fM and so allows for a direct assessment within different sample preparation conditions. The reason this large range is because the dynamic range necessary to measure the full range of expression in cells is the biological range of expression, which is typically three orders of magnitude. A normalisation factor is calculated as follows:
   i) For each of the experimental samples, the raw counts of the six positive controls are added up, giving N1, N2, N3 etc;
   ii) the average of these data points is calculated;
   iii) a normalisation factor is determined for each sample column by dividing the average value from (ii) by N1 for sample 1, N2 for sample 2, N3 for sample 3 etc from (i);
   iv) the calculated normalisation factor should be between 0.3 and 3 (recommendations from Nanostring). If the factor is outside of this range, the normalisation of the count data may not reflect the actual concentration of the control targets and care must be taken when interpreting results from such assays. The miRNAs of the present invention had a normalisation factor between 0.65 - 1.26; and
   v) a new column is created for the normalised data corresponding to each column of un-normalised data. Each sample count value is multiplied by the normalisation factor for that column, giving a normalised expression value.
3. Use of negative controls - each assay includes 8 negative controls for which no target sequence is present in the human transcriptome. They can therefore be used to estimate systematic background counts within any single hybridisation reaction. The median value of these 8 controls was taken as the first cut-off value: miRNAs with counts below this median value were excluded from further analysis. Therefore, we had normalised data with values above this cut-off.
4. Demonstration of differential expression of the miRNAs from Tamoxifen Resistant vs Tamoxifen-Revertant cells - miRNAs were only selected if the average count of the miRNAs from the 4 Tamoxifen-Resistant experiments demonstrated at least a 1.2-fold difference over the average counts from the Tamoxifen-Revertant cells.
5. Fold-difference from Wild Type -the chosen normalised miRNAs then had to demonstrate a minimum of a 1.2 fold difference in counts compared to the Wild Type samples, either up- or down.
6. Selection of only those miRNAs which demonstrated concordance between the Tam-Resistant and Tam-Revertant samples, i.e. if the miRNA count was increased in the Tam-Resistant cells, it could not show a greater increase in the Tam-Revertant cells and if the miRNA count was reduced in the Tam-Resistant cells there could not be a greater reduction in the Tam-Revertants. This indicated biological relevance of our chosen miRNAs.

This protocol resulted in the inventors identifying the miRNAs listed in Tables 1 and 2.

### Example 2

The biological significance of the miRNAs of the invention listed in Tables 1 and 2 can be exemplified by reference to regulation of PTEN expression. PTEN is one of the most commonly lost tumour suppressors in human cancer and its loss is associated with poor prognosis in breast cancer. Seven of the selected up-regulated miRNAs, including miR-26a and miR-26b, have been shown to be direct regulators of PTEN expression *(*Huse JT, et a.,IGenes Dev. 2009 Jun 1;23(11):1327-37 *and* Palumbo T et al., Oncogene.2012 May 21*).* The PTEN pathway has an important regulatory function in TR and reduced PTEN expression predicts relapse in breast cancer patients treated with tamoxifen. PTEN is a negative regulator of PI3K/Akt pathway, and, together with PI3K/Akt pathway activators HER2 and IGF1R, controls normal cell growth. If this pathway is inappropriately activated, cells grow in an uncontrolled manner. Trastuzumab (herceptin) is an antibody that binds selectively to the HER2 protein and prevents it from activating the PI3K/Akt pathway. This increases the survival of people with cancer. Reduced expression of PTEN stops Herceptin-mediated growth arrest of HER2-overexpressing breast cancer cells and is associated with a much poorer response to trastuzumab-based therapy than in those with normal PTEN.

Promoter methylation of this gene has already been shown to be associated with TR. The present inventors predict that whilst treatment with a DNMT inhibitor can increase expression of PTEN, a complete restoration of its activity will require additional therapy using miR antagonists. Furthermore, the inventors predict that Herceptin-unresponsiveness in a significant percentage of breast cancers may also be caused by aberrant miRNA expression.

### Example3

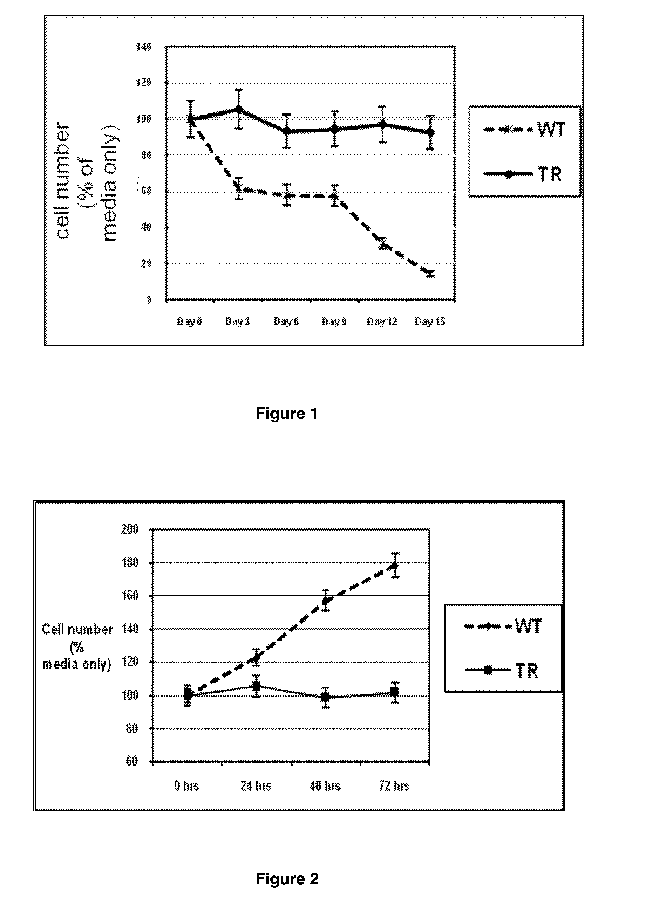

Two tamoxifen-resistant "TR" breast cancer cells lines were developed by subjecting cells from the MCF-7 breast cancer cell line to long-term (>9 months) repeated re-incubation in the presence of high dose tamoxifen (10⁻⁷M). Subsequent comparison between these tamoxifen-resistant cells with wild type (WT) cells of the growth effects of tamoxifen confirmed their resistance (see Figure 1), as did their incubation with estradiol showing absence of the normal oestrogen-induced growth stimulation (see Figure 2).

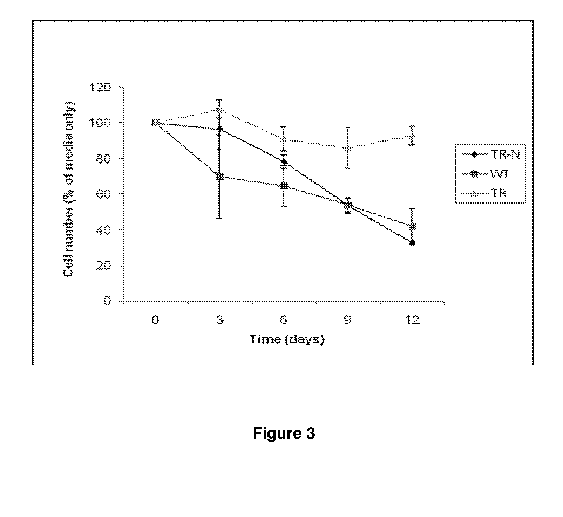

Subsequently, both clones of the TR cells were repeatedly re-incubated every 5-7 days, or when confluent within the incubation flask for more than six months in the presence of ideal concentrations of estrogen (10⁻⁹M) without tamoxifen. This resulted in the development of cells that regained sensitivity to the growth inhibitory effects of tamoxifen (TRev) (see Figure 3). These cell lines offer a unique opportunity to elucidate the mechanisms of acquired tamoxifen resistance.

The establishment of these two cell lines demonstrating restoration of tamoxifen sensitivity in cells which were previously resistant to its effects fundamentally alters the generally accepted view of the mechanisms responsible for tamoxifen resistance. These findings demonstrate that it cannot be the result of genetic mutations, which would be irreversible, but must be due to a reversible epigenetic effect.

### Example 4

The expression levels of all currently known miRNAs in the three cell lines was first determined:
(1) wild type (WT) tamoxifen-sensitive MCF7 cells;
(2) tamoxifen-resistant (TR) cells derived from (1); and
(3) tamoxifen sensitive revertant (TRev) cells derived from (2).

This was done using a novel and highly sensitive technology, NanoStrinng Counter Analysis. This digital method can detect a single copy of RNA and can evaluate up to 800 targets per reaction. Unlike microarrays or PCR-based gene expression analysis technologies, this method does not use nor is its performance limited by enzymes; hence the dynamic range necessary to measure the full range of expression in cells is simply the biological range of expression, typically three orders of magnitude. Results obtained using this technology are not necessarily comparable to results obtained using miRNA arrays or RT-qPCR-based methods.

Data analysis involves the use of both positive and negative controls, which ensures its stringent accuracy. The system can measure precise changes in expression of a single transcript.

### Experimental Results

### [A] UP-REGULATED miRNAs:

### WT vs TR cells

Table 1 shows miRNAs that are up-regulated in TR cells by at least 1.2-fold compared with WT. miR-141 has also been found not to be detectable in WT cells but highly expressed in TR cells.

### TR vs TRev cells

An association of any of these up-regulated miRNAs with TR would be strengthened if the TRev cells had significantly reduced, or WT levels of that miRNA. This is shown to be the case in Table 3. Clearly, the levels of most miRNAs in the TRev cells are significantly below those of the TR cells. Furthermore, the miRNAs that are most up-regulated in TR cells are also the ones whose expression is the most reduced in the TRev cells.

**Table 3**

| **miRNA** | **Relative Expression Rev:TR** |
|---|---|
| hsa-miR-21 | 0.2 |
| hsa-miR-16 | 0.5 |
| hsa-miR-26a | 0.3 |
| hsa-miR-141 | 0.2 |
| hsa-miR-15a | 0.4 |
| hsa-miR-15b | 0.6 |
| hsa-miR-24 | 0.7 |
| hsa-miR-26b | 0.7 |
| hsa-miR-103 | 0.6 |
| hsa-miR-191 | 0.6 |
| hsa-miR-520d-5p | 0.3 |
| hsa-miR-527 | 0.3 |
| hsa-miR-518a-5p | 0.3 |
| hsa-miR-30b | 0.8 |
| hsa-miR-30c | 0.7 |
| hsa-miR-200a | 0.6 |
| hsa-miR-361-5p | 0.5 |
| hsa-miR-770-5p | 0.8 |
| hsa-miR-106b | 0.4 |
| hsa-miR-96 | 0.6 |
| hsa-miR-30d | 0.6 |
| hsa-miR-301a | 0.4 |
| hsa-miR-425 | 0.7 |
| hsa-miR-455-3p | 0.8 |

### [B] DOWN-REGULATED miRNAs

### WT vs TR cells

Table 2 shows miRNAs that are down-regulated in TR cells by at least 1.2-fold compared to WT.

### TR vs TRev cells

Analogous to the situation with the increased miRNAs, an association of any of these down-regulated miRNAs with TR would be strengthened if the TRev cells had significantly increased, or WT levels of that miRNA. As shown in Table 4, most of the miRNAs are expressed at significantly higher levels in the TRev cells compared with the TR cells.

**Table 4**

| **miRNA** | **Relative Expression Rev:TR** |
|---|---|
| hsa-let-7d | 2.2 |
| hsa-let-7a | 2.5 |
| hsa-miR-548h | 1.4 |
| hsa-miR-365 | 1.6 |
| hsa-miR-93 | 2.1 |
| hsa-miR-620 | 1.3 |
| hsa-miR-548j | 1.4 |
| hsa-miR-31 | 1.3 |
| hsa-miR-181b | 1.4 |
| hsa-miR-181d | 1.4 |
| hsa-miR-450a | 1.6 |
| hsa-miR-302f | 1.4 |
| hsa-let-7c | 2.1 |
| hsa-miR-660 | 1.4 |
| hsa-miR-218 | 1.4 |
| hsa-miR-188-5p | 1.3 |
| hsa-miR-892a | 1.2 |
| hsa-miR-320a | 2.0 |
| hsa-miR-720 | 1.4 |
| hsa-miR-181a | 2.7 |
| hsa-miR-645 | 1.7 |
| hsa-miR-572 | 1.4 |
| hsa-miR-518f | 1.3 |
| hsa-miR-1289 | 1.2 |
| hsa-miR-649 | 1.5 |
| hsa-miR-324-5p | 1.3 |
| hsa-miR-1299 | 1.3 |
| hsa-miR-378 | 1.2 |
| hsa-miR-335 | 1.3 |
| hsa-miR-592 | 1.9 |
| hsa-miR-302c | 1.4 |
| hsa-miR-526a | 1.6 |
| hsa-miR-518d-5p | 1.6 |
| hsa-miR-520c-5p | 1.6 |
| hsa-miR-1279 | 1.5 |
| hsa-miR-1182 | 1.3 |
| hsa-miR-503 | 1.5 |
| hsa-miR-222 | 1.4 |
| hsa-miR-1283 | 1.5 |
| hsa-miR-320d | 1.4 |
| hsa-miR-555 | 1.3 |
| hsa-miR-1537 | 1.4 |
| hsa-miR-664 | 1.5 |
| hsa-miR-519e | 1.5 |
| hsa-miR-136 | 1.3 |
| hsa-miR-580 | 1.2 |
| hsa-miR-492 | 1.7 |
| hsa-miR-495 | 1.6 |
| hsa-miR-525-3p | 1.7 |
| hsa-miR-383 | 1.6 |
| hsa-miR-615-5p | 1.5 |
| hsa-miR-875-3p | 1.3 |
| hsa-miR-488 | 1.4 |
| hsa-miR-1298 | 1.4 |
| hsa-miR-450b-5p | 1.6 |
| hsa-miR-484 | 1.9 |
| hsa-miR-876-5p | 1.2 |
| hsa-miR-363 | 1.4 |
| hsa-miR-1909 | 1.4 |
| hsa-miR-514 | 1.4 |
| hsa-miR-655 | 1.2 |
| hsa-miR-1277 | 1.4 |
| hsa-miR-1973 | 1.3 |
| hsa-miR-34c-3p | 1.2 |
| hsa-miR-142-5p | 1.5 |
| hsa-miR-410 | 1.2 |
| hsa-miR-2115 | 1.5 |
| hsa-miR-515-3p | 1.4 |
| hsa-miR-661 | 1.5 |
| hsa-miR-548g | 1.7 |
| hsa-miR-767-5p | 1.5 |
| hsa-miR-412 | 1.5 |
| hsa-miR-146a | 1.2 |
| hsa-miR-574-5p | 1.8 |
| hsa-miR-490-5p | 1.4 |
| hsa-miR-362-5p | 1.5 |
| hsa-miR-641 | 1.2 |
| hsa-miR-548f | 1.4 |
| hsa-miR-328 | 1.5 |
| hsa-miR-663 | 1.5 |
| hsa-miR-337-5p | 1.6 |
| hsa-miR-1321 | 1.3 |
| hsa-miR-208b | 1.5 |
| hsa-miR-10b | 1.8 |
| hsa-miR-708 | 1.3 |
| hsa-miR-491-3p | 1.3 |
| hsa-miR-619 | 1.6 |
| hsa-miR-375 | 14.0 |
| hsa-miR-190b | 1.7 |
| hsa-miR-1206 | 1.3 |
| hsa-miR-423-5p | 1.7 |
| hsa-miR-566 | 1.7 |
| hsa-miR-193a-5p | 1.5 |
| hsa-miR-453 | 1.4 |
| hsa-miR-302b | 1.3 |
| hsa-miR-496 | 1.3 |
| hsa-miR-623 | 1.4 |
| hsa-miR-99b | 1.8 |
| hsa-miR-590-3p | 1.5 |
| hsa-miR-1287 | 1.4 |
| hsa-miR-1268 | 1.2 |
| hsa-miR-553 | 1.3 |
| hsa-miR-651 | 1.5 |
| hsa-miR-608 | 1.3 |
| hsa-miR-125a-5p | 1.9 |
| hsa-miR-1979 | 1.6 |
| hsa-miR-34b | 1.4 |
| hsa-miR-208a | 1.4 |
| hsa-miR-7 | 1.3 |
| hsa-miR-206 | 1.2 |
| hsa-miR-561 | 1.3 |
| hsa-miR-609 | 1.3 |
| hsa-miR-576-5p | 1.6 |
| hsa-miR-197 | 1.5 |
| hsa-miR-1308 | 1.5 |
| hsa-miR-33a | 1.3 |
| hsa-miR-127-5p | 1.4 |
| hsa-miR-635 | 1.6 |
| hsa-miR-548c-5p | 1.6 |
| hsa-miR-1915 | 1.8 |
| hsa-miR-499-5p | 1.3 |
| hsa-miR-760 | 1.4 |
| hsa-miR-510 | 1.2 |
| hsa-miR-600 | 1.6 |
| hsa-m i R-199a-3p | 1.5 |
| hsa-miR-199b-3p | 1.5 |
| hsa-miR-554 | 1.5 |
| hsa-miR-938 | 1.8 |
| hsa-miR-137 | 1.3 |
| hsa-miR-629 | 1.3 |
| hsa-miR-603 | 2.3 |
| hsa-miR-769-5p | 1.5 |
| hsa-miR-1825 | 1.6 |
| hsa-let-7e | 3.0 |
| hsa-miR-1255a | 1.5 |
| hsa-miR-595 | 1.4 |
| hsa-miR-1908 | 1.8 |
| hsa-miR-216a | 1.9 |
| hsa-let-7b | 4.8 |
| hsa-miR-542-3p | 2.5 |
| hsa-miR-193a-3p | 1.9 |
| hsa-miR-205 | 4.2 |

### Conclusions

We have used a digital analysis technology to identify miRNAs whose expression is significantly altered in TR cells and, crucially, whose expression resembles the wild type (tamoxifen sensitive) pattern when those cells revert to a wild type phenotype.

## Claims

1. An *ex vivo* method for determining whether tumour cells in a patient are, or are likely to become, resistant to a drug that modulates the binding of a steroid hormone to its receptor, comprising:
(i) determining the expression level of one or more of the miRNAs listed in Table 1 and/or Table 2 in a tumour cell sample obtained from the patient; and
(ii) comparing the expression level of each miRNA to a corresponding control (WT) value, wherein an increase in the cellular expression level of one or more miRNAs listed in Table 1 or a decrease in the cellular expression level of one or more of the miRNAs listed in Table 2 indicates that the tumour cells are, or are likely to become, resistant to the drug, preferably wherein the tumour cell is a breast cancer cell.

2. A method according to claim 1, wherein the drug is a selective estrogen receptor modulator (SERM), preferably tamoxifen.

3. A method according to claim 1 or claim 2, wherein an increase or decrease in cellular expression of at least +/-1.2-fold compared to the control is indicative of drug resistance.

4. A method according to any preceding claim, wherein the patient has previously been treated using a drug that modulates the binding of a steroid hormone to its receptor, preferably tamoxifen.

5. A method according to any of claims 1 to 3, wherein the method is carried out prior to administering anti-cancer therapy to the patient.

6. An agent that decreases cellular expression of at least one miRNA listed in Table 1 or increases cellular expression of at least one miRNA listed in Table 2, for use in reverting tamoxifen-resistant tumour cells to a tamoxifen-sensitive phenotype for the treatment of breast cancer.

7. An agent according to claim 6, preferably selected from the group comprising miRNA or anti-miRNA molecules and drugs which prevent DNA methylation and/or histone modification, such as 5-azacytidine, 5-aza-2'-deoxycytidine, phenylbutyrate, sodium butyrate, valproic acid, suberoylanilidehydroxamic acid and dispeptide.

8. A therapeutic regimen for use in the treatment of a hormone-sensitive cancer, comprising the steps of:
(i) administering a first drug that modulates the binding of a steroid hormone to its receptor to a patient suffering from a hormone-sensitive cancer;
(ii) determining when cancerous cells of the patient become, or are close to becoming, resistant to said first drug by carrying out the method according to any of claims 1 to 3;
(iii) administering a second drug, wherein the second drug is an agent that prevents DNA methylation and/or histone modification and/or decreases cellular expression of one or more of the miRNAs listed in Table 1 and/or increases cellular expression of one or more of the miRNAs listed in Table 2; and
(iv) ceasing to administer the second drug after a pre-determined period of time, or after establishing reversion to a drug-sensitive phenotype,
wherein said regimen prevents the cancerous cells from becoming permanently resistant to said first drug.

9. A therapeutic regimen according to claim 7, wherein the steroid hormone is preferably estrogen and the receptor is the estrogen receptor, and wherein the first drug is a selective estrogen receptor modulator, preferably tamoxifen, and wherein the second drug is preferably selected from the group comprising miRNA or anti-miRNA molecules and drugs whichprevent DNA methylation and/or histone modification, such as 5-azacytidine, 5-aza-2'-deoxycytidine, phenylbutyrate, sodium butyrate, valproic acid, suberoylanilidehydroxamic acid and dispeptide.

10. A therapeutic regimen according to claim 7 or claim 8, wherein the first drug is withdrawn whilst said second drug is administered, and preferably wherein the first drug is re-administered following step (iv).

11. An ex vivomethod for determining whether tumour cells previously identified as being resistant to a drug that modulates the binding of a steroid hormone to its receptor have reverted to a drug-sensitive phenotype, comprising
(i) identifying one or more of the miRNAs listed in Table 1 and/or Table 2 that was previously known to be over- or under-expressed in a tumour cell obtained from a patient having a drug-resistant phenotype;
(ii) determining the expression level of the one or more of the miRNAs identified in step (i) in a tumour cell sample obtained from the patient; and either
(iii) comparing the expression level to a corresponding control (WT) value, wherein a value within less than +/-1.2-fold of the controlvalue indicates that the cell has reverted to a drug-sensitive phenotype; or
(iv) comparing the expression level to the expression level of the corresponding miRNA as previously measured in the patient sample known to be drug-resistant, wherein a decrease of at least 1.2-fold in the cellular expression level of one or more miRNAs listed in Table 1 and/or an increase of at least 1.2-foldin the cellular expression level of one or more of the miRNAs listed in Table 2 indicates that the tumour cells have reverted from a resistant to sensitive phenotype,
wherein the drug is preferably a selective estrogen receptor modulator (SERM), preferably tamoxifen.

12. A method according to claim 11, wherein a change in cellular expression level of at least +/-1.2-fold compared to the control or previous expression level measured in a drug-resistant cell is indicative of reversion to a drug-sensitive phenotype.

13. A biochip comprising oligonucleotide sequences specific to each of one or more of the miRNAs listed in Table 1 and/or Table 2, preferably wherein the biochip can detect the expression level of five or more of the miRNAs listed in Table 1 and/or 2, more preferably up to ten, more preferably up to twenty, and most preferably all of the miRNA molecules listed in Table 1 and 2.

14. Use of a biochip according to claim 13, to determine whether tumour cells in a patient sample have become, or are likely to become, resistant to tamoxifen therapy, or to determine whether previously resistant tumour cells have reverted to a tamoxifen-sensitive phenotype.

15. An *in vitro* method for restoring a drug-sensitive phenotype in cells of a drug-resistant cell line, said method comprising repeatedly re-incubating the cells in the absence of the drug to which the cells are resistant, preferably also in the presence of a steroid hormone, preferably wherein the steroid hormone is estrogen, and preferably wherein the estrogen concentration is 10⁻⁹M, preferably wherein the drug-resistant cell line is a hormone-sensitive cancer cell line, preferably a tamoxifen-resistant cell line, preferably derived from a tamoxifen-sensitive cell line, and most preferably derived from the MCF-7 cell line.
